# EUROPEAN PATENT SPECIFICATION

(11) **EP 1 366 080 B1**
(45) Date of publication and mention of the grant of the patent: **29.11.2006**
(21) Application number: 02727364.8
(22) Date of filing: 06.03.2002
(51) Int. Cl.: C07K 16/42, A61K 39/395

(54) **NATURAL ANTIBODIES ACTIVE AGAINST HIV VIRUS**
NATÜRLICHER ANTIKÖRPER MIT WIRKUNG GEGEN DAS HIV VIRUS
ANTICORPS NATURELS ACTIFS CONTRE LE VIRUS DU VIH

(30) Priority: 09.03.2001 IT MI20010500; 31.10.2001 IT MI20012285
(43) Date of publication of application: 03.12.2003
(73) Proprietor: Diapharm Limited, St. Peter Port, Guernsey, Channel Islands GY1 4EJ (GB)
(72) Inventor: METLAS, Radmila, Guernsey GY1 4EJ, Channel Islands (GB)
(74) Representative: Minoja, Fabrizio
(86) International application number: PCT/EP2002/002454
(87) International publication number: WO 2002/072637

(56) References cited:
- METLAS R ET AL: "Anti-V3 and anti-IgG antibodies of healthy individuals share complementarity structures." JOURNAL OF ACQUIRED IMMUNE DEFICIENCY SYNDROMES (1999) UNITED STATES 1 AUG 1999, vol. 21, no. 4, 1 August 1999 (1999-08-01), pages 266-270, XP002205581 ISSN: 1525-4135
- METLAS R ET AL: "Human immunodeficiency virus V3 peptide-reactive antibodies are present in normal HIV-negative sera." AIDS RESEARCH AND HUMAN RETROVIRUSES. UNITED STATES 1 MAY 1999, vol. 15, no. 7, 1 May 1999 (1999-05-01), pages 671-677, XP002205582 ISSN: 0889-2229
- FUNG M ET AL: "Monoclonal anti-idiotypic antibody mimicking the principal neutralization site in HIV-1 gp120 induces HIV-1 neutralizing antibodies in rabbits" JOURNAL OF IMMUNOLOGY, THE WILLIAMS AND WILKINS CO. BALTIMORE, US, vol. 145, no. 7, 1 October 1990 (1990-10-01), pages 2199-2206, XP002125226 ISSN: 0022-1767
- METLAS R ET AL: "Does the HIV-1 manipulate immune network via gp120 immunoglobulin-like domain involving V3 loop?" VACCINE, BUTTERWORTH SCIENTIFIC. GUILDFORD, GB, vol. 13, no. 4, 1995, pages 355-359, XP004057732 ISSN: 0264-410X

## Description

The present invention relates to the use of antibodies neutralizing the Human Immunodeficiency Virus. Said antibodies are present in sera from normal subjects and can be used to prevent the HIV infection or to delay the onset of illness in serum positive patients. The present invention further relates to pharmaceutical preparations containing said antibodies and the use thereof in passive immunotherapy of HIV infection.

### BACKGROUND OF THE INVENTION

The induction of effective immune response to HIV-1 has often been restricted by the inability of any vaccine candidate to elicit antibodies capable of neutralizing infectivity of primary HIV isolates from infected individuals (1,2). On the other hand, from the analysis of sera from infected subjects resulted that neutralization of primary virus tends to be weak and sporadic (3,4). However, albeit it is difficult to raise antibodies, either during the infection or with experimental vaccines, monoclonal antibodies exist, such as IgG1b12, 2F5 and 2G12 (5-7), which are capable of neutralizing primary viruses of subtypes A - E. Said antibodies derive from subtype B - infected individuals and they are not raised by vaccination (8).

The earliest detectable HIV-1 specific antibody is of the IgG isotype (12), suggesting a nonconventional primary response (13). Furthermore, there are findings indicating that HIV-1 antigens react with pre-existing, antibody repertoires (14). Cross-reactive antibodies that recognize the HIV-1 envelope have been reported (15).

It was suggested that a strong early humoral response in HIV-1 infection may not be beneficial (16) as it would result in monoclonal and polyclonal antibody population instead of normal polyclonal response (13). It has been suggested that anti-idiotypic antibody 1F7 may broaden immune response to HIV antigens (17).

In our earlier studies, the homology of HIV envelope protein with IgG variable protein was predicted (18,19). However, recent data suggest that HIV-1 gp120 immunodominant epitope and a fraction of naturally-occurring IgG antibodies share complementary structure (20.21).

### DISCLOSURE OF THE INVENTION

It has now been found that a group of antibodies present in sera from normal, not HIV - infected individuals, are capable of neutralizing HIV virus.

The antibody active fraction was purified from sera of normal subjects by affinity chromatography using a Sepharose resin to which total human IgG had been coupled. Observation evidenced that such anti-IgG antibodies fraction is capable of neutralizing HIV-1 infection in PBMC. The experiments were repeated using different anti-IgG preparations of various concentrations, obtained from commercial serum specimens, using affinity purified total IgG antibodies as negative control and HIVIG and mAb 4117C antibodies as positive control. The latter proved to be inactive to 92HT593B. Neutralization has been demonstrated in a representative experiment carried out with HIV-1 primary isolates 92HT593B and SF162WT as well as with NLHX-ADA recombinant virus. Anti-IgG antibodies proved to be active against 92HT593B and NLHX-ADA at a 50% inhibiting concentration ranging from less than 1 to 7 µg/ml.

There is little possibility for the neutralizing effects observed with the preparation of anti-IgG antibodies to be due to co-purified chemokines from normal sera, as the concentration of these substances does not exceed 20 ng per ml of serum and no specific bands were revealed by PAGE. Furthermore, molecules below 50kDa were removed by dialysis of the antibodies preparations. PAGE analysis of anti-IgG preparations revealed predominant presence of IgG and very small traces of contaminant proteins.

A possible explanation of the observed effects is that HIV-1 antigenic determinants share complementary structure with the variable regions of natural antibodies belonging to the immune network (9,23), but in no way this or other explanations limit the invention.

Therefore, the invention relates to the use of a preparation of human antibodies raised against the total human IgG fraction, capable of neutralizing HIV-1. Said preparation is obtained subjecting sera from normal, not HIV-infected subjects, to affinity chromatography using resins coupled to total human IgG fraction. The final concentration of isolated anti-IgG antibodies will be preferably comprised within the range of 0.1-1000 µg/ml, more preferably within the range of 0.1-100 µg/ml. The starting material consists of a pool of sera from normal subjects. According to a further aspect, the invention allows to the use of the preparation of the invention in the prophylactic or therapeutical treatment of HIV infection, preferably HIV-1 infection.

For the use in therapy, the preparation of the invention will be suitably formulated with pharmaceutically acceptable excipients. The latter include buffering agents, stabilizing agents, solubilizers, diluents, isotonicity agents and the like. The preparation will preferably be administered parenterally, preferably through the intravenous, intradermal or intramuscular route. According to a preferred embodiment, the preparation will be used in passive immunotherapy of HIV-1 infection.

### EXAMPLE 1 - Preparation of anti-IgG antibodies

Anti-IgG antibodies were prepared from a pool of two normal human sera by affinity chromatography using Sepharose beads (CNBr-activated Sepharose 4B) to which total human IgG (purified on GammaBind Sepharose 4B affinity column) had previously been coupled. In principle, 5-7 mg of IgG antibodies / 0.5-0.6 g of Sepharose beads were used. Binding was carried out following the indications of the manufacturer. The column was equilibrated with 5xPBS and heat-inactivated serum (1ml) was loaded and diluted to 1:1 by 5xPBS. Incubation was carried out for 1 hour at room temperature or overnight at 4°C. After washing with 30 ml of 5xPBS, bound antibodies were eluted using 0.1 M citrate, pH=2.5 into test-tubes containing base-TRIS. The collected eluate was concentrated, dialyzed (Centricon YM, 30000 MW cut-off) and analyzed through 10% SDS-PAGE, predominantly revealing the IgG band (Fig. 2).

### EXAMPLE 2 - Neutralization of HIV-1 isolates by anti-IgG antibodies

Virus neutralization was evaluated in PBMC following a previously published protocol (22). Neutralization assays were carried out with four different preparations of antibodies in five separated experiments.

Table 1 summarizes neutralization data against two HIV primary isolates (92HT593B, SF162WT) and a recombinant NL-HX-ADA virus.

HIVIG and mAb4117C were used as positive controls; Protein-G IgG was used as negative control.

In this test, all of the preparations of anti-IgG antibodies proved capable of inhibiting PBMC infection by 92HT593B and NL-HX-ADA, whereas no neutralizing activity against SF162WT was observed.

**Table: Viral neutralization titers of purified Ig from sera from HIV-1 infected (a) and normal (b) subjects**

| | | Primary isolate | | | | | Recombinant virus | |
|---|---|---|---|---|---|---|---|---|
| | | 92HT593B | | | SF162WT | | NLHX-ADA | |
| | | | µg/ml | % neut | µg/ml | % neut | µg/ml | % neut. |
| Antibodies Preparat. Assay | | | | | | | | |
| Negative control: | | | | | | | | |
| Protein G IgG b) | | | 150 | 8.3 | 150 | 2.9 | 150 | -6.8 |
| | | | 50 | 1.1 | 50 | -0.5 | 50 | -4.4 |
| | | | 16.7 | 2.2 | 16.7 | 2.7 | 16.7 | -4.4 |
| Positive control: | | | | | | | | |
| mAb4117C a) | | | 100 | 16.1 | 100 | 100 | 100 | 98.7 |
| | | | 33.3 | 9.0 | 33.3 | 100 | 33.3 | 96.0 |
| | | | 11.1 | 5.2 | 11.1 | 100 | 1.1 | 55.0 |
| | | | 3.7 | 0.4 | 3.7 | 80.4 | 3.7 | 23.2 |
| | | | 1.2 | -0.6 | 1.2 | 57.4 | 1.2 | -8.6 |
| Positive control: | | | | | | | | |
| HIVIG a) | | | 100 | 88.0 | 100 | 57.4 | 100 | 93.4 |
| | | | 33.3 | 62.0 | 33.3 | 39.9 | 33.3 | 94.8 |
| | | | 11.1 | 44.4 | 11.1 | 2.1 | 11.1 | 92.4 |
| | | | 3.7 | 39.4 | 3.7 | -14.4 | 3.7 | 87.2 |
| | | | 1.23 | 22.2 | 1.23 | 0.7 | 1.23 | 69.2 |
| | | | 0.41 | 8.3 | 0.41 | -3.7 | 0.41 | 54.5 |
| Anti-IgG b) | 1 | 1 | 1.87 | 57.4 | | | | |
| | | | 0.62 | 37.6 | NA | | ND | |
| | | | 0.21 | 6.1 | | | | |
| Anti-IgG b) | 1 | 2 | 1.23 | 71.8 | | | | |
| | | | 0.41 | 48.9 | NA | | ND | |
| | | | 0.14 | 14.4 | | | | |
| Anti-IgG b) | 2 | 1 | 8.00 | 42.0 | | | 8.00 | 60.3 |
| | | | 2.67 | 24.1 | NA | | 2.67 | 14.6 |
| | | | 0.89 | 5.7 | | | 0.89 | 1.3 |
| Anti-IgG b) | 3 | 1 | 2.17 | 46.4 | | | | |
| | | | 0.72 | 27.1 | | NA | ND | |
| | | | 0.24 | 3.3 | | | | |
| Anti-IgG b) | 4 | 1 | 10.97 | 39.8 | | | 10.97 | 68.7 |
| | | | 3.66 | - | NA | | 3.66 | 54.0 |
| | | | 1.22 | - | | | 1.22 | 45.0 |

### LITERATURE

1. Mascola, JR, Snyder, SW, Weislow, OS. Belay, SM, Belshe, RB, Schwartz, DH, Clements, ML, Dolin, R, Graham, BS, Gorse, GJ, Keefer, MC, McElrath, MJ, Walker, MC, Wagner, KF, McNeil, JG, McCutchan, FE, and Burke, DS: Immunization with envelope subunit vaccine products elicits neutralizing antibodies against laboratory-adapted but not primary isolates of human immunodeficiency virus type 1. J Infect Dis 1996;173:340-348.
2. Montefiori, DC, and Evans, TG: Toward and HIV type 1 vaccine that generates potent, broadly cross-reactive neutralizing antibodies. AIDS Res Hum Retroviruses 1999;15:689-698;
3. Moore, JP, Coa, Y, Leu, Qin, L, Korber, B, and Ho, DD: Inter and intraclade neutralization of human immunodeficiency virus type 1: genetic clades do not correspond to neutralization serotypes but partially correspond to gp120 antigenic serotypes. J Virol 1996;70:427-444;
4. Weber, J, Fenyo, EM, Beddows, S, Kaleeby, P, Bjorndal, A, and the WHO Network for HIV Isolation and Characterization: Neutralization serotypes of HIV-1 field isolates are not predicted by genetic subtype. J Virol 1996;70:7827-7832;
5. Burton, DR, Pyati, J, Koduri, R, Sharp, SJ, Thornton, GB, Parren, PWHI, Sawyer, LSW, Hendry, RM, Dunlop, N, Nara, PL, Lamacchia, M, Garratty, E, Stiehm, ER, Bryson, YJ, Cao, Y, Moore, JO, Ho, DD, and Barbas III, CF: Efficient neutralization of primary isolates of HIV-1 by a recombinant human monoclonal antibody. Science 1994;266:1024-1027;
6. Mascola, JR, Louder, MK, VanCott, TC, Sapan, CV, Lambert, JS, Muenz, LR, Bunow, B, Birx, DL, and Robb, ML: Potent and synergistic neutralization of human immunodeficiency virus (HIV) type 1 primary isolates by hyperimmune anti-HIV immunoglobulin combined with monoclonal antibodies 2F5 and 2G12. J Virol 1997;71:7198-7206;
7. Trkola, A, Pomales, AB, Yuan, H, Kober, B, Maddon, PJ, Allaway, GP, Katinger, H, Barbas III, CF, Burton, DR, Ho, DD, and Moore, JP: Cross-clade neutralization of primary isolates of human immunodeficiency virus type 1 by human monoclonal antibodies and tetrameric CD4-IgG. J Virol 1995;69:6609-6617;
8. Moore, JP, and Trkola, A: JIV type 1 coreceptors, neutralization serotypes, and vaccine development. AIDS Res Human Retroviruses 1997;13:733-736;
9. Coutinho A: Beyond clonal selection and network. Immunological Reviews 1989;110:63-67;
10. Avrames, S: Natural autoantibodies: from "horror autotexicus" to "gnothi seauton". Immunol Today 1991;12:154-159;
11. Moller, G: Effect of anti-immunoglobulin sera on B lymphocyte functions. Scand J Immunol 1978;8:469-474;
12. Race, EM, Ramsey, KM, Lucia, HL, and Cloyd, MW: Human immunodeficiency virus elicits antibody not detected by standard tests; implications for diagnostics and viral immunology. Virology 1991;184:716-722;
13. Nara, PL, and Garrity, R: Deceptive imprinting: a cosmopolitan strategy for complicating vaccination. Vaccine 1998;16:1780-17787;
14. Zubler, RH, Perrin, LH, Doucet, A, Zhang, X, Huang, YP, and Miescher, PA: Frequencies of HIV-reactive B cells in seropositive and seronegative individuals. Clin Exp Immunol 1991; 87:31-36;
15. Davis, D, Chaudhri, B, Stephens, DM, Came, CA, Willers, C, and Lachmann, PJ: The immunodominance of epitopes within the transmembrane protein (gp41) of human immunodeficiency virus type 1 may be determined by the host's previous exposure to similar epitopes on unrelated antigens. J Gen Virol 1990;71:1975-1983;
16. Velijovic, V, Metlas, R, Köhler, H, Umovitz, HB, Prljic, J, Velikovic, N, Johnson, E, and Müller, S: AIDS epidemic at the beginning of the third millennium: time for a new AIDS vaccine strategy. Vaccine 2000;19:1855-1863;
17. Wang, QL, Wang, HT, Blalock, E, Moller, S, and K"hlerm H: Identification of an idiotypic peptide recognized by autoantibodies in immunodeficiency virus-1-infected individuals. Idiotype-specific autoantibodies in AIDS 1995;96:775-780;
18. Veljkovic, V, and Metlas, R: Identification of immunoglobulin recombination elements in HIV-1 envelope gene. Immunol Lett 1991;31:11-14;
19. Metlas, R, Veljkovic, V, Paladini, DR, and Pongor, S: Protein and DNA sequence homology between the V3 loop of human immunodeficiency virus type 1 envelope protein gp120 and immunoglobulin variable region. Biochem Biophys Res Commun 1991;179:1056-1062;
20. Metlas, R. Skerl, V, Veljkovic, V, Colombatti, A, and Pongor, S: Immunoglobulin-like domain of HIV-1 envelope glycoprotein gp120 encodes putative internal image of some common human proteins. Viral Immunol 1994;7:215-219;
21. Metlas, R, Trajkovic, D, Srdic, T, Veljkovic, V, and Colombatti, A: Anti-V3 and anti-IgG antibodies of normal individuals share complementariry structures. JAIDS 1999;21:266-270;
22. Pinter, A, Honnen, WJ, Tilley, SA: Conformational changes affecting the V3 and CD4-binding domains of human immunodeficiency virus type 1 gp120 associated with env processing and with binding of ligands to these sites. J Virol 1993;67:5692-5697;
23. Holmberg, D, Andersson, A, Carlsson, L, and Forsgren, S: Establishment and functional implications of B-cell connectivity. Immunological Reviews 1989;110:89-103;
24. Ochsenbein, AF, and Zinkernagel, RM: Natural antibodies and complement link innate and acquired immunity. Immunol Today 2000;21:624-629;
25. Fearon, DT, and Locksley, RM: The instructive role of innate immunity in the acquired immune response. Science 1996;272:50-54.

## Claims

1. The use of human antibodies against human IgG total fraction, wherein said human antibodies are obtained by affinity chromatography of sera from normal, HIV-uninfected individuals, using resins coupled to human IgG total fraction, and are capable of neutralising HIV virus, for the manufacture of a medicament for passive immunotherapy of HIV-1 infections.

## Patentansprüche

1. Die Verwendung von Humanantikörpern gegen die Human-IgG-Gesamtfraktion, worin die besagten Humanantikörper durch Affinitätschromatographie der Sera von normalen nicht-HIVinfizierten Individuen erhalten werden, unter Verwendung von Harzen, die an die Human-IgG-Gesamtfraktion gekoppelt sind, und fähig sind, das HIV-Virus zu neutralisieren, für die Herstellung eines Medikaments für die passive Immuntherapie von HIV-1-Infektionen.

## Revendications

1. Utilisation d'anticorps humains contre une fraction totale d'IgG humaine, dans laquelle lesdits anticorps humains sont obtenus par chromatographie par affinité de sérums auprès de personnes normales non infectées par le VIH, en utilisant des résines couplées à une fraction totale d'IgG humaine, et sont capables de neutraliser le virus VIH, pour la fabrication d'un médicament pour l'immunothérapie passive d'infections dues au VIH -1.
